(19) Europäisches Patentamt
European Patent Office
Office européen des brevets

(11) Veröffentlichungsnummer: **0 437 415 B1**

(12) **EUROPÄISCHE PATENTSCHRIFT**

(45) Veröffentlichungstag der Patentschrift: **02.08.95**

(51) Int. Cl.$^6$: **C07D 471/04**, A61K 31/435

(21) Anmeldenummer: **91810003.3**

(22) Anmeldetag: **03.01.91**

(54) **Kristallform des 4-(5,6,7,8-Tetrahydroimidazo[1,5-a]pyridin-5-yl)-benzonitril-hydrochlorids.**

(30) Priorität: **12.01.90 CH 100/90**

(43) Veröffentlichungstag der Anmeldung:
**17.07.91 Patentblatt 91/29**

(45) Bekanntmachung des Hinweises auf die Patenterteilung:
**02.08.95 Patentblatt 95/31**

(84) Benannte Vertragsstaaten:
**AT BE CH DE DK ES FR GB GR IT LI LU NL SE**

(56) Entgegenhaltungen:
**EP-A- 0 165 904**

(73) Patentinhaber: **CIBA-GEIGY AG**
**Klybeckstrasse 141**
**CH-4002 Basel (CH)**

(72) Erfinder: **Ibrahim, Jutta, Dr.**
**Lindenstrasse 96**
**CH-4433 Ramlinsburg (CH)**

EP 0 437 415 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine neue kristallwasserhaltige Kristallform des 4-(5,6,7,8-Tetrahydroimidazo[1,5-a]pyridin-5-yl)-benzonitril-hydrochlorids der Formel Ia,

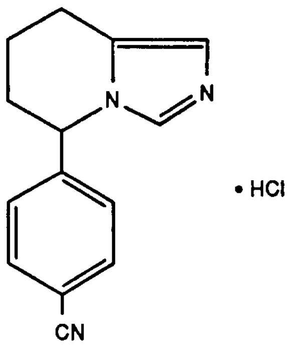

(Ia)

Verfahren zur Herstellung derselben, pharmazeutische Präparate, die diese enthalten und ihre Verwendung zur therapeutischen Behandlung des menschlichen oder tierischen Körpers oder zur Herstellung pharmazeutischer Präparate.

Die Verbindung der Formel Ia wird zum Beispiel in der europäischen Patentanmeldung Nr. 165 904 als "5-(p-Cyanphenyl)-5,6,7,8-tetrahydroimidazo[1,5-a]pyridin-Hydrochlorid" offenbart und aufgrund ihrer das Enzym Aromatase hemmenden Eigenschaften u.a. zur Behandlung von Oestrogen-abhängigen Krankheiten, insbesondere Oestrogen-abhängigem Brustkrebs bei postmenopausalen Frauen, vorgeschlagen.

In der erwähnten Patentanmeldung wird auch die Herstellung der Verbindung der Formel Ia beschrieben. Sie erfolgt in den meisten Fällen durch Lösen der freien Base in wenig Aceton und Zugabe einer etherischen HCl-Lösung. In einem Beispiel wird auch die Herstellung der Verbindung der Formel Ia direkt durch Cyclisierung von 4-[4-Chlor-4-(p-cyanphenyl)-n-butyl]-1H-imidazol gelöst in Chloroform beschrieben. In allen Fällen wird dabei die Verbindung der Formel Ia in wasserfreier Form, als Anhydrat, erhalten.

Es wurde nun gefunden, dass die Kristalle des Anhydrats der Formel Ia in mindestens zwei verschiedenen Kristallmodifikationen vorliegen können. Die eine, im folgenden "α-Anhydrat" genannt, ist durch die folgenden Gitterabstände (d-Werte) und relativen Linienintensitäten (Intensität) ihres Röntgenpulverdiagramms charakterisiert:

| d-Werte [in Angstroem (≙ $10^{-10}$ m) | Intensität |
|---|---|
| 9,5 | sehr stark |
| 6,8 | sehr schwach |
| 6,6 | sehr stark |
| 5,89 | mittel |
| 5,10 | stark |
| 4,87 | mittel |
| 4,75 | schwach |
| 4,61 | stark |
| 4,30 | sehr schwach |
| 4,19 | sehr stark |
| 4,01 | mittel |
| 3,79 | mittel |
| 3,61 | mittel |
| 3,58 | mittel |
| 3,47 | sehr stark |
| 3,37 | sehr schwach |
| 3,30 | sehr stark |
| 3,23 | sehr schwach |
| 3,03 | sehr schwach |

Ueber die Messmethodik ist folgendes zu sagen (Diese Angaben gelten in gleicher Weise für alle weiter unten offenbarten Röntgenpulverdiagramme): Zur Bestimmung der Gitterabstände = Netzebenenabstände (d-Werte) wird das Beugungsbild auf Film registriert. Die Aufnahme erfolgt in Transmission mit einer Guinierkamera (Enraf-Nonius FR 552) und Kupfer-$K_{\alpha1}$-Strahlung (Wellenlänge $\lambda$ = 1,54060 Angstroem). Zur Eichung wird ein auf dem gleichen Film aufgenommenes Diagramm von Quarz benutzt. Die relativen Linienintensitäten werden durch Prüfung mit dem Auge abgeschätzt.

Die zweite Kristallmodifikation des Anhydrats der Formel Ia, im folgenden als "$\beta$-Anhydrat" bezeichnet, ist durch das folgende Röntgenpulverdiagramm charakterisiert:

| d-Werte [in Angstroem (≙ $10^{-10}$ m) | Intensität |
|---|---|
| 12,1 | sehr stark |
| 7,3 | schwach |
| 6,6 | sehr stark |
| 6,0 | sehr stark |
| 5,93 | sehr schwach |
| 5,83 | stark |
| 4,61 | stark |
| 4,53 | mittel |
| 4,46 | stark |
| 4,31 | mittel |
| 4,13 | stark |
| 4,03 | mittel |
| 3,99 | schwach |
| 3,94 | mittel |
| 3,66 | schwach |
| 3,51 | sehr stark |
| 3,48 | stark |
| 3,40 | stark |
| 3,34 | stark |
| 3,28 | mittel |
| 3,21 | stark |
| 3,11 | mittel |
| 3,01 | schwach |
| 2,97 | mittel |

Die Kristalle des Anhydrats der Formel Ia - sowohl das $\alpha$- als auch das $\beta$-Anhydrat - weisen den Nachteil auf, dass sie hygroskopisch sind, d.h. in Abhängigkeit von der Umgebungsfeuchtigkeit wechselnde Wassermengen aufnehmen. Auch aus relativ trockener Luft, etwa mit einer relativen Feuchtigkeit von 33 % oder 44 %, wird Wasser adsorbiert (s. Tabellen 1 und 2).

Tabelle 1

| Wasseraufnahme des $\alpha$-Anhydrats der Formel Ia bei 44 % relativer Feuchtigkeit und T = 23 °C (im Klimaglas) in Abhängigkeit von der Zeit | |
|---|---|
| Zeit [Tage] | Wassergehalt [in %]*) |
| 0 | 0,62 |
| 4 | 2,43 |
| 7 | 2,82 |
| 11 | 3,12 |
| 15 | 3,19 |
| 18 | 3,28 |
| 21 | 3,32 |

*) Basis: Trockengewicht des Anhydrats

Tabelle 2

| Wasseraufnahme des α-Anhydrats der Formel Ia bei 33 % relativer Feuchtigkeit und T = 23 °C (im Klimaglas) in Abhängigkeit von der Zeit | |
|---|---|
| Zeit [Tage] | Wassergehalt [in %]*) |
| 0 | 0,62 |
| 4 | 0,75 |
| 7 | 0,85 |
| 11 | 1,00 |

*) Basis: Trockengewicht des Anhydrats

Aufgrund der Tatsache, dass die Kristalle des Anhydrats der Formel Ia hygroskopisch sind, ist ihre Lagerstabilität in starkem Masse beeinträchtigt. Folglich wird ihre Verarbeitung zu pharmazeutischen Präparaten, insbesondere zu für die orale Verabreichung geeigneten Darreichungsformen, wie Tabletten, erschwert.

Ferner existieren vom Anhydrat der Formel Ia mindestens zwei verschiedene Kristallmodifikationen. Dies kompliziert die Herstellung pharmazeutischer Präparate, insbesondere von Festpräparaten, da man ständig mit einer - partiellen oder vollständigen - Umwandlung der einen Modifikation in die andere rechnen bzw. Vorkehrungen treffen muss, eine solche Umwandlung zu verhindern. Verschiedene Kristallmodifikationen können unterschiedliche Eigenschaften aufweisen, z.B. hinsichtlich ihrer Mikronisierbarkeit, ihrer generellen Tablettiereigenschaften oder ihrer Löslichkeit. Verwendet man versehentlich anstelle einer vorgesehenen Kristallmodifikation X eine andere Kristallmodifikation Y, die sich aus X gebildet hat, oder ein Gemisch aus X und Y, so können ernste Probleme bei der Herstellung pharmazeutischer Präparate auftreten.

Aufgabe der vorliegenden Erfindung ist es daher, eine neue Kristallform des 4-(5,6,7,8-Tetrahydroimidazo[1,5-a]pyridin-5-yl)-benzonitril-hydrochlorids bereitzustellen, welche die obengenannten Nachteile nicht aufweist und insbesondere auch bei mehrjähriger Lagerung stabil ist.

Es wurde nun eine neue Kristallform des 4-(5,6,7,8-Tetrahydroimidazo[1,5-a]pyridin-5-yl)-benzonitrilhydrochlorids gefunden, welche überraschenderweise die dem Anhydrat der Formel Ia innewohnenden Nachteile nicht aufweist und bei normalen Umgebungsbedingungen völlig lagerstabil ist. Es handelt sich hierbei um die Kristallform des 4-(5,6,7,8-Tetrahydroimidazo[1,5-a]pyridin-5-yl)benzonitril-hydrochlorid-hemihydrats der Formel I:

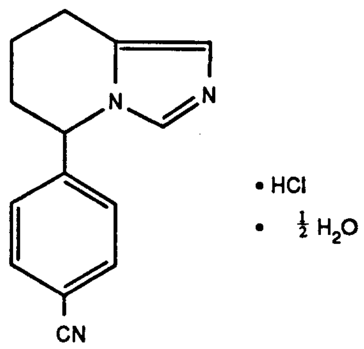

(I)

Es wurde festgestellt, dass überraschenderweise weder eine Variation der relativen Feuchtigkeit im Bereich von etwa 10 % bis etwa 75 % noch 8-tägiges Erhitzen auf 35 ° bzw. 50 °C das Röntgenpulverdiagramm oder den Wassergehalt der Kristalle des Hemihydrats der Formel I nachweisbar verändert (s. Tabelle 3).

Tabelle 3

| Wasseradsorptions- und Wasserdesorptionsversuche mit dem Hemihydrat der Formel I | | | | |
|---|---|---|---|---|
| Bedingungen | Dauer [Tage] | T [°C] | relative Feuchtigkeit [%] | Wassergehalt [%][1)] |
| Klimaglas | [2)] | 23 | 75 (NaCl)[3)] | 3,74 |
| Klimaglas | [2)] | 23 | 66 ($Na_2CrO_4$)[3)] | 3,59 |
| Klimaglas | [2)] | 23 | 54 ($Na_2Cr_2O_7$)[3)] | 3,54 |
| Klimaglas | [2)] | 23 | 33 ($MgCl_2$)[3)] | 3,54 |
| Klimaglas | [2)] | 23 | 23 ($CH_3COOK$)[3)] | 3,59 |
| Klimaglas | [2)] | 23 | 12 (LiCl)[3)] | 3,54 |
| offenes Gefäss | 8 | 35 | (≙ 21 %) | 3,54 |
| offenes Gefäss | 8 | 50 | (≙ 10 %) | 3,44 |

[1)] Basis: Trockengewicht des Anhydrats; theoretischer Wert für das Hemihydrat der Formel I: 3,47 %
[2)] nach Gleichgewichtseinstellung
[3)] Klimaeinstellung über entsprechenden gesättigten, wässrigen Lösungen

Aus der Tabelle 3 geht hervor, dass die erfindungsgemässen Kristalle des Hemihydrats der Formel I uneingeschränkt lagerstabil sind. Dies bedeutet, dass sie als Massenware ("Los" bzw. "Batch") über Jahre gelagert werden können, ohne sich zu verändern. Insbesondere nehmen sie kein Wasser mehr auf, so dass der Wirkstoffgehalt bei der Lagerung konstant bleibt und sich nicht - wie beim bekannten Anhydrat der Formel Ia - ständig verringert bei gleicher Gewichtseinwaage. Dies ist insbesondere von Bedeutung im Hinblick auf die ausserordentlich hohe Wirksamkeit der Verbindung und die damit zusammenhängende niedrige Dosierung am Menschen, die im Bereich von lediglich einem oder wenigen Milligramm liegt (s. unten).

Die Kristalle des Hemihydrats der Formel I sind durch die folgenden Gitterabstände (d-Werte) und relativen Linienintensitäten (Intensität) ihres Röntgenpulverdiagramms charakterisiert:

| d-Werte [in Angstroem (≙ $10^{-10}$ m) | Intensität |
|---|---|
| 13,4 | sehr stark |
| 9,5 | mittel |
| 8,2 | schwach |
| 7,5 | stark |
| 6,7 | sehr schwach |
| 6,5 | sehr stark |
| 5,65 | sehr stark |
| 5,42 | stark |
| 5,34 | sehr stark |
| 5,15 | mittel |
| 4,99 | mittel |
| 4,84 | stark |
| 4,60 | stark |
| 4,51 | stark |
| 4,20 | stark |
| 4,09 | mittel |
| 4,00 | stark |
| 3,83 | stark |
| 3,81 | mittel |
| 3,76 | schwach |
| 3,74 | mittel |
| 3,66 | mittel |
| 3,62 | stark |
| 3,56 | mittel |
| 3,51 | mittel |
| 3,40 | sehr schwach |
| 3,35 | mittel |
| 3,29 | sehr schwach |
| 3,25 | stark |
| 3,21 | schwach |
| 3,19 | schwach |
| 3,15 | mittel |
| 3,13 | sehr schwach |
| 3,04 | stark |
| 3,01 | schwach |

Die Kristalle des Hemihydrats der Formel I sind ferner durch ihre Elementaranalyse charakterisiert, die im Einklang mit den für die Summenformel $C_{14}H_{13}N_3 \cdot HCl \cdot \frac{1}{2}H_2O$(MG: 268,75) berechneten Werten steht: C 62,57 %; H 5,63 %; N 15,64 %; Cl 13,19 %.

Bevorzugt betrifft die Erfindung die Kristalle des Hemihydrats der Formel I in im wesentlichen reiner Form.

Die Kristalle des Hemihydrats der Formel I werden in an sich bekannter Weise z.B. dadurch hergestellt, dass man

(a) das 4-(5,6,7,8-Tetrahydroimidazo[1,5-a]pyridin-5-yl-benzonitril mit Chlorwasserstoff in Gegenwart von Wasser umsetzt, oder

(b) eine Erscheinungsform des 4-(5,6,7,8-Tetrahydroimidazo[1,5-a]pyridin-5-yl)-benzonitril-hydrochlorids, die weniger Wasser als das herzustellende Hemihydrat der Formel I enthält, mit Wasser behandelt, oder

(c) einer Erscheinungsform des 4-(5,6,7,8-Tetrahydroimidazo[1,5-a]pyridin-5-yl)-benzonitril-hydrochlorids, die mehr Wasser als das herzustellende Hemihydrat der Formel I enthält, Wasser entzieht, oder

(d) ein vom Hydrochlorid verschiedenes Salz des 4-(5,6,7,8-Tetrahydroimidazo[1,5-a]pyridin-5-yl)-benzonitrils in Gegenwart von Wasser in das Hydrochlorid-Hemihydrat der Formel I überführt.

Verfahren (a): Die als Ausgangsmaterial dienende freie Base 4-(5,6,7,8-Tetrahydroimidazo[1,5-a]pyridin-5-yl)-benzonitril wird vor der Umsetzung vorzugsweise in einem organischen Lösungsmittel, z.B. Ethylmethylketon oder insbesondere Aceton, gelöst.

Vorzugsweise wird die freie Base mit einer wässrigen Chlorwasserstofflösung in das Hydrochlorid-Hemihydrat der Formel I überführt. Die Konzentration der wässrigen Chlorwasserstofflösung ist an sich

nicht kritisch. Um jedoch die Ausbeuteverluste möglichst gering zu halten, ist die Verwendung von konzentrierter Salzsäure, insbesondere 37%-iger Salzsäure, bevorzugt.

Die Reaktionstemperatur liegt z.B. zwischen 0° und 70°C und vorteilhaft zwischen 20° und 55°C; in erster Linie wird die Umsetzung bei Raumtemperatur durchgeführt.

Verfahren (b): Die Behandlung von insbesondere festen Erscheinungsformen des 4-(5,6,7,8-Tetrahydroimidazo[1,5-a]pyridin-5-yl)-benzonitril-hydrochlorids, die wasserärmer als das erfindungsgemässe Hemihydrat der Formel I sind, z.B. dem oben erwähnten α- oder β-Anhydrat der Formel Ia, mit Wasser erfolgt in üblicher Weise durch Einwirkung mindestens der für die Bildung des Hemihydrats erforderlichen Wassermenge.

Das Wasser liegt dabei vorzugsweise im flüssigen oder gasförmigen Aggregatzustand vor.

Bei der Behandlung mit Wasser im flüssigen Aggregatzustand wird die wasserarme Erscheinungsform des 4-(5,6,7,8-Tetrahydroimidazo[1,5-a]pyridin-5-yl)-benzonitril-hydrochlorids in Wasser oder einem wässrigen Lösungsmittelgemisch suspendiert oder gelöst und dann die Kristalle des Hemihydrats der Formel I in üblicher Weise isoliert. Für den Fall, dass es sich um eine Lösung handelt, muss das Hemihydrat der Formel I zunächst in üblicher Weise zur Kristallisation gebracht werden, z.B. durch Einengen der Lösung, d.h. Verdampfung eines Teils der Lösung, Hinzufügen eines mit Wasser mischbaren Lösungsmittels, in dem das Hemihydrat der Formel I weniger gut löslich ist als in Wasser, oder durch Temperaturerniedrigung. In einer bevorzugten Ausführungsform wird die Kristallisation des Hemihydrats der Formel I durch Animpfen mit Impfkristallen des Hemihydrats der Formel I ausgelöst bzw. beschleunigt.

Bei der Behandlung mit Wasser im gasförmigen Aggregatzustand setzt man das Ausgangsmaterial einer wasserdampfhaltigen Atmosphäre, z.B. feuchter Luft, aus. Die für die Umwandlung erforderliche Expositionsdauer nimmt mit zunehmender relativer Feuchtigkeit ab. Bei Raumtemperatur beträgt die relative Feuchtigkeit bevorzugt etwa 50 bis 70 %.

Die Isolierung des Produkts geschieht in üblicher Weise, etwa durch Zentrifugieren, Filtration oder Druckfiltration ("Abnutschen"). Die Trocknung des Produkts geschieht vorteilhaft bei 20°-30°C; bevorzugt ist eine Vakuumtrocknung bei 20°C.

Verfahren (c): Als Erscheinungsformen des 4-(5,6,7,8-Tetrahydroimidazo[1,5-a]pyridin-5-yl)-benzonitril-hydrochlorids kommen insbesondere solche, die fest oder flüssig sind, in Frage, z.B. feuchte Kristalle, wässrige Suspensionen, wässrige Lösungen, Suspensionen in wässrigen Lösungsmittelgemischen oder Lösungen in wässrigen Lösungsmittelgemischen.

Der Entzug des überschüssigen Wassers erfolgt z.B. durch Trocknung, vorteilhaft bei 20-30°C und vorzugsweise im Vakuum bei 20°C.

Handelt es sich um Lösungen oder Suspensionen, werden die Kristalle des Hemihydrats der Formel I vor der Trocknung vorzugsweise isoliert, z.B. wie beim Verfahren (b) angegeben durch Kristallisation und/oder Zentrifugieren, Filtration oder Druckfiltration. Man kann die Lösungen bzw. Suspensionen jedoch auch direkt am Vakuum, z.B. bei 20-30°C, eindampfen.

Verfahren (d): Ein vom Hydrochlorid verschiedenes Salz ist vorzugsweise ein pharmazeutisch annehmbares Salz.

Das Verfahren (d) ist an sich bekannt; beispielsweise wird das vom Hydrochlorid verschiedene Salz in einem wasserhaltigen Lösungsmittel oder Lösungsmittelgemisch gelöst und mit einem Ueberschuss an Chloridionen oder insbesondere Chlorwasserstoff versetzt. Vorzugsweise wird das Lösungsmittel so gewählt, dass das Hydrochlorid darin eine geringere Löslichkeit hat als das Salz, von dem man ausgegangen ist.

Das als Ausgangsstoff benötigte vom Hydrochlorid verschiedene Salz wird z.B. durch Umsetzung der freien Base 4-(5,6,7,8-Tetrahydioimidazo[1,5-a]pyridin-5-yl)-benzonitril mit der entsprechenden Säure hergestellt.

Die vorliegende Erfindung betrifft ferner pharmazeutische Präparate, die als Wirkstoff die Kristalle des Hemihydrats der Formel I enthalten. Besonders bevorzugt sind Präparate zur enteralen, insbesondere oralen, Verabreichung. Die Präparate enthalten den Wirkstoff allein oder vorzugsweise zusammen mit einem oder mehreren pharmazeutisch verwendbaren Trägermaterialien. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 0,1 % bis etwa 40 % des Wirkstoffs, wobei einzeldosierte Applikationsformen vorzugsweise von etwa 0,1 % bis etwa 20 % und nicht-einzeldosierte Applikationsformen vorzugsweise etwa 0,1 % bis etwa 10 % Wirkstoff aufweisen. Dosiseinheitsformen, wie Dragees, Tabletten oder Kapseln, enthalten von etwa 0,1 mg bis etwa 20 mg, vorzugsweise etwa 1 mg bis etwa 4 mg, des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragee-Kernen verarbeitet

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat.

Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung Von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Die Erfindung betrifft ebenfalls ein Verfahren zur Behandlung von Krankheiten bei Säugern einschliesslich Menschen, die auf eine Hemmung der Aktivität des Enzyms Aromatase bzw. eine Unterdrückung der Oestrogensynthese ansprechen, z.B. Oestrogenabhängige Krankheiten, insbesondere Oestrogen-abhängige Tumoren, z.B. Oestrogenabhängiger Brustkrebs, oder Gynäkomastie. Dabei kann das Hemihydrat der vorliegenden Erfindung prophylaktisch oder therapeutisch verabreicht werden. Bei einem Warmblüter mit einem Körpergewicht von etwa 70 kg wird eine tägliche Dosis von etwa 0,1 mg bis etwa 20 mg, vorzugsweise von etwa 1 mg bis etwa 4 mg, des Hemihydrats der vorliegenden Erfindung verabreicht.

Die nachfolgenden Beispiele illustrieren die vorliegende Erfindung; sie sollen jedoch diese in ihrem Umfang in keiner Weise einschränken. Temperaturen werden in Grad Celsius angegeben.

Beispiel 1: 4-(5,6,7,8-Tetrahydroimidazo[1,5-a]pyidin-5-yl)-benzonitril-hydrochlorid-hemihydrat

In eine gerührte Vorlage von 200 l Aceton werden bei 20-25° 33 kg 4-(5,6,7,8-Tetrahydroimidazo[1,5-a]pyridin-5-yl-benzonitril eingetragen. Sobald alles gelöst ist, werden 1,56 l Wasser sowie 1,8 kg Aktivkohle, angeschlämmt in 6 l Aceton, zugegeben. Nach 45 min Rühren bei 20-25° wird der Ansatz filtriert und der Rückstand mit 138 l Aceton gewaschen. Zu den vereinigten Filtraten lässt man unter Rühren bei 20-25° ca. 12,5 l Salzsäure (37 %) zulaufen, bis pH 2,5-1,0 erreicht ist. Die so erhaltene Suspension wird 3 h bei 20-25° nachgerührt, zentrifugiert und der Rückstand in Portionen mit insgesamt 80 l Aceton gewaschen. Nach Vakuumtrocknung bei 20° erhält man die Titelverbindung als weisses, kristallines Pulver; Röntgenpulverdiagramm: Gitterabstände/Intensitäten wie oben angegeben; IR (Nujol): 3430 (stark), 3375 (stark), 2225 (stark) $cm^{-1}$;

| Elementaranalyse: $C_{14}H_{13}N_3 \cdot HCl \cdot \frac{1}{2} H_2O$ (MG: 268,75) | | | | |
|---|---|---|---|---|
| Berechnet: | C 62,57 %; | H 5,63 %; | N 15,64 %; | Cl 13,19 % |
| Gefunden: | C 62,53 %; | H 5,57 %; | N 15,66 %; | Cl 13,21 % |

Wasserbestimmung (nach Karl-Fischer):
Berechnet: 3,35 %[1)]
Gefunden: 3,43 %.

Die als Ausgangsmaterial dienende freie Base wird wie in EP-A-165 904 beschrieben hergestellt, insbesondere durch Ringschluss von 5-(3-Chlorpropyl)-1-(p-cyanphenylmethyl)-1H-imidazol mit Kalium-tert.-butoxid in Tetrahydrofuran (s. Beispiel 1 in EP-A-165 904).

Beispiel 2: 4-(5,6,7,8-Tetrahydroimidazo[1,5-a]pyridin-5-yl)-benzonitril-hydrochlorid-hemihydrat

In ein gerührtes Gemisch bestehend aus 1,5 l Wasser und 4,2 l Aceton werden 5,3 kg 4-(5,6,7,8-Tetrahydroimidazo[1,5-a]pyridin-5-yl)-benzonitril-hydrochlorid ($\alpha$-Anhydrat) eingetragen. Man heizt auf und rührt bei 50-55°, bis eine klare Lösung vorliegt. Dann kühlt man die Lösung auf 30° ab und impft sie mit 5 g 4-(5,6,7,8-Tetrahydroimidazo[1,5-a]pyridin-5-yl)-benzonitril-hydrochlorid-hemihydrat. Nach dem Einsetzen der Kristallisation wird das Gemisch innerhalb von 45 min auf 0° abgekühlt. Bei 0-5° werden innerhalb von 1 h 35 l Aceton zudosiert. Die Suspension wird 2 h bei 0-5° nachgerührt, zentrifugiert und der kristalline Rückstand mit 8 l Aceton gewaschen. Nach dem Trocknen im Vakuum bei 20° erhält man die Titelverbindung;
Röntgenpulverdiagramm: Gitterabstände/Intensitäten wie oben angegeben;
IR (Nujol): 3430 (stark), 3375 (stark), 2225 (stark) $cm^{-1}$;

| Elementaranalyse: $C_{14}H_{13}N_3 \cdot HCl \cdot \frac{1}{2}H_2O$ (MG: 268,75) | | | | |
|---|---|---|---|---|
| Berechnet: | C 62,57 %; | H 5,63 %; | N 15,64 %; | Cl 13,19 % |
| Gefunden: | C 62,63 %; | H 5,58 %; | N 15,67 %; | Cl 13,25 % |

Wasserbestimmung (nach Karl-Fischer):
Berechnet: 3,35 %
Gefunden: 3,46 %.

Das als Ausgangsmaterial dienende $\alpha$-Anhydrat wird wie in EP-A-165 904 beschrieben hergestellt, nämlich durch Lösen der freien Base in wenig Aceton und Neutralisation mit etherischem Chlorwasserstoff (s. Beispiel 1 in EP-A-165 904).

Beispiel 3: Es werden 10000 Tabletten à 100 mg hergestellt, die je 2 mg Wirkstoff enthalten:

Zusammensetzung:

| | |
|---|---|
| 4-(5,6,7,8-Tetrahydroimidazo[1,5-a]pyridin-5-yl)-benzonitril-hydrochlorid-hemihydrat | 20,70 g |
| Siliciumdioxid kolloidal | 2,00 g |
| Cellulose mikrokristallin | 100,00 g |
| Lactose, sprühgetrocknet | 817,30 g |
| Magnesiumstearat | 10,00 g |
| Natriumcarboxymethylcellulose | 50,00 g |
| | 1000,00 g |

Alle Bestandteile des Tablettenkerns werden miteinander vermischt. Sobald eine homogene Mischung erreicht ist, wird diese zu Tablettenkernen verpresst.

[1)] Der hier berechnete theoretische Wassergehalt (basierend auf dem Molekulargewicht des Hemihydrats der Formel I) steht nicht im Widerspruch zum theoretischen Wassergehalt des Hemihydrats der Formel I in Tabelle 3, welcher auf dem Trockengewicht (= Molekulargewicht) des Anhydrats basiert.

Beispiel 4: Es werden 10000 Tabletten à 100 mg hergestellt, die je 1 mg Wirkstoff enthalten:

Zusammensetzung:

| | |
|---|---|
| 4-(5,6,7,8-Tetrahydroimidazo[1,5-a]pyridin-5-yl)-benzonitril-hydrochlorid-hemihydrat | 10,35 g |
| Lactose kristallin | 739,65 g |
| Cellulose mikrokristallin | 230,00 g |
| Siliciumdioxid kolloidal | 10,00 g |
| Magnesiumstearat | 10,00 g |
| | 1000,00 g |

Alle Bestandteile des Tablettenkerns werden miteinander vermischt. Sobald eine homogene Mischung erreicht ist, wird diese zu Tablettenkernen verpresst.

**Patentansprüche**

**Patentansprüche für folgende Vertragsstaaten : AT, BE, CH, DE, DK, FR, GB, IT, LI, LU, NL, SE**

1. 4-(5,6,7,8-Tetrahydroimidazo[1,5-a]pyridin-5-yl)-benzonitril-hydrochlorid in kristalliner Hemishydratform.

2. 4-(5,6,7,8-Tetrahydroimidazo[1,5-a]pyridin-5-yl)-benzonitril-hydrochlorid in kristalliner Hemihydratform gemäss Anspruch 1, gekennzeichnet durch die folgenden Gitterabstände (d-Werte) und relativen Linienintensitäten (Intensität) im Röntgenpulverdiagramm (Guinierkamera, Kupfer-$K_{\alpha 1}$-Strahlung):

| d-Werte [in Angstroem (≙ $10^{-10}$ m) | Intensität |
|---|---|
| 13,4 | sehr stark |
| 9,5 | mittel |
| 8,2 | schwach |
| 7,5 | stark |
| 6,7 | sehr schwach |
| 6,5 | sehr stark |
| 5,65 | sehr stark |
| 5,42 | stark |
| 5,34 | sehr stark |
| 5,15 | mittel |
| 4,99 | mittel |
| 4,84 | stark |
| 4,60 | stark |
| 4,51 | stark |
| 4,20 | stark |
| 4,09 | mittel |
| 4,00 | stark |
| 3,83 | stark |
| 3,81 | mittel |
| 3,76 | schwach |
| 3,74 | mittel |
| 3,66 | mittel |
| 3,62 | stark |
| 3,56 | mittel |
| 3,51 | mittel |
| 3,40 | sehr schwach |
| 3,35 | mittel |
| 3,29 | sehr schwach |
| 3,25 | stark |
| 3,21 | schwach |
| 3,19 | schwach |
| 3,15 | mittel |
| 3,13 | sehr schwach |
| 3,04 | stark |
| 3,01 | schwach |

**3.** Die Kristallform gemäss Anspruch 1 in im wesentlichen reiner Form.

**4.** Die Kristallform gemäss Anspruch 2 in im wesentlichen reiner Form.

**5.** Pharmazeutisches Präparat enthaltend 4-(5,6,7,8-Tetrahydroimidazo[1,5-a]pyridin-5-yl)-benzonitril-hydrochlorid in einer Kristallform gemäss Anspruch 1 oder 2 und mindestens ein pharmazeutisch verwendbares Trägermaterial.

**6.** Die Kristallform gemäss einem der Ansprüche 1 bis 4 zur Anwendung in einem Verfahren zur therapeutischen Behandlung des tierischen oder menschlichen Körpers.

**7.** Die Kristallform gemäss einem der Ansprüche 1 bis 4 zur Verwendung bei der Behandlung von Krankheiten, die auf eine Hemmung des Enzyms Aromatase ansprechen.

**8.** Verwendung einer Kristallform gemäss einem der Ansprüche 1 bis 4 zur Herstellung pharmazeutischer Präparate.

**9.** Verwendung einer Kristallform gemäss einem der Ansprüche 1 bis 4 zur Herstellung pharmazeutischer Präparate für die Behandlung von Krankheiten, die auf eine Hemmung des Enzyms Aromatase

ansprechen.

**10.** Verfahren zur Herstellung einer Kristallform gemäss einem der Ansprüche 1 bis 4, dadurch gekennzeichnet, dass man
(a) das 4-(5,6,7,8-Tetrahydroimidazo[1,5-a]pyridin-5-yl)-benzonitril mit Chlorwasserstoff in Gegenwart von Wasser umsetzt, oder
(b) eine Erscheinungsform des 4-(5,6,7,8-Tetrahydroimidazo[1,5-a]pyridin-5-yl)-benzonitril-hydrochlorids, die weniger Wasser als das herzustellende Hemihydrat der Formel I enthält, mit Wasser behandelt, oder
(c) einer Erscheinungsform des 4-(5,6,7,8-Tetrahydroimidazo[1,5-a]pyridin-5-yl)-benzonitril-hydrochlorids, die mehr Wasser als das herzustellende Hemihydrat der Formel I enthält, Wasser entzieht, oder
(d) ein vom Hydrochlorid verschiedenes Salz des 4-(5,6,7,8-Tetrahydroimidazo[1,5-a]pyridin-5-yl)-benzonitrils in Gegenwart von Wasser in das Hydrochlorid-Hemihydrit der Formel I überführt.

**Patentansprüche für folgende Vertragsstaaten : ES, GR**

**1.** Verfahren zur Herstellung von 4-(5,6,7,8-Tetrahydroimidazo[1,5-a]pyridin-5-yl)-benzonitril-hydrochlorid in kristalliner Hemihydratform, dadurch gekennzeichnet, dass man
(a) das 4-(5,6,7,8-Tetrahydroimidazo[1,5-a]pyridin-5-yl)-benzonitril mit Chlorwasserstoff in Gegenwart von Wasser umsetzt, oder
(b) eine Erscheinungsform des 4-(5,6,7,8-Tetrahydroimidazo[1,5-a]pyridin-5-yl)-benzonitril-hydrochlorids, die Weniger Wasser als das herzustellende Hemihydrat der Formel I enthält, mit Wasser behandelt, oder
(c) einer Erscheinungsform des 4-(5,6,7,8-Tetrahydroimidazo[1,5-a]pyridin-5-yl)-benzonitril-hydrochlorids, die mehr Wasser als das herzustellende Hemihydrat der Formel I enthält, Wasser entzieht, oder
(d) ein vom Hydrochlorid verschiedenes Salz des 4-(5,6,7,8-Tetrahydroimidazo[1,5-a]pyridin-5-yl)-benzonitrils in Gegenwart von Wasser in das Hydrochlorid-Hemihydrat der Formel I überführt.

**2.** Verfahren gemäss Anspruch 1 zur Herstellung von 4-(5,6,7,8-Tetrahydroimidazo[1,5-a]pyridin-5-yl)-benzonitril-hydrochlorid in kristalliner Hemihydratform mit folgenden Gitterabständen (d-Werte) und relativen Linienintensitäten (Intensität) im Röntgenpulverdiagramm (Guinierkamera, Kupfer-$K_{\alpha 1}$-Strahlung):

| d-Werte [in Angstroem (≙ $10^{-10}$ m) | Intensität |
|---|---|
| 13,4 | sehr stark |
| 9,5 | mittel |
| 8,2 | schwach |
| 7,5 | stark |
| 6,7 | sehr schwach |
| 6,5 | sehr stark |
| 5,65 | sehr stark |
| 5,42 | stark |
| 5,34 | sehr stark |
| 5,15 | mittel |
| 4,99 | mittel |
| 4,84 | stark |
| 4,60 | stark |
| 4,51 | stark |
| 4,20 | stark |
| 4,09 | mittel |
| 4,00 | stark |
| 3,83 | stark |
| 3,81 | mittel |
| 3,76 | schwach |
| 3,74 | mittel |
| 3,66 | mittel |
| 3,62 | stark |
| 3,56 | mittel |
| 3,51 | mittel |
| 3,40 | sehr schwach |
| 3,35 | mittel |
| 3,29 | sehr schwach |
| 3,25 | stark |
| 3,21 | schwach |
| 3,19 | schwach |
| 3,15 | mittel |
| 3,13 | sehr schwach |
| 3,04 | stark |
| 3,01 | schwach |

**3.** Verfahren gemäss Anspruch 1 zur Herstellung der Kristallform gemäss Anspruch 1 in im wesentlichen reiner Form.

**4.** Verfahren gemäss Anspruch 2 zur Herstellung der Kristallform gemäss Anspruch 2 in im wesentlichen reiner Form.

**Claims**
**Claims for the following Contracting States : AT, BE, CH, DE, DK, FR, GB, IT, LI, LU, NL, SE**

**1.** 4-(5,6,7,8-Tetrahydroimidazo[1,5-a]pyridin-5-yl)-benzonitrile hydrochloride in crystalline hemihydrate form.

**2.** 4-(5,6,7,8-Tetrahydroimidazo[1,5-a]pyridin-5-yl)-benzonitrile hydrochloride in crystalline hemihydrate form according to claim 1, the X-ray powder pattern of which (Guinier camera, copper-$K_{\alpha 1}$ radiation) exhibits the following lattice spacings (d values) and relative line intensities (intensity):

| d values [in Angstrom (≙ $10^{-10}$ m)] | Intensity |
|---|---|
| 13.4 | very strong |
| 9.5 | medium |
| 8.2 | weak |
| 7.5 | strong |
| 6.7 | very weak |
| 6.5 | very strong |
| 5.65 | very strong |
| 5.42 | strong |
| 5.34 | very strong |
| 5.15 | medium |
| 4.99 | medium |
| 4.84 | strong |
| 4.60 | strong |
| 4.51 | strong |
| 4.20 | strong |
| 4.09 | medium |
| 4.00 | strong |
| 3.83 | strong |
| 3.81 | medium |
| 3.76 | weak |
| 3.74 | medium |
| 3.66 | medium |
| 3.62 | strong |
| 3.56 | medium |
| 3.51 | medium |
| 3.40 | very weak |
| 3.35 | medium |
| 3.29 | very weak |
| 3.25 | strong |
| 3.21 | weak |
| 3.19 | weak |
| 3.15 | medium |
| 3.13 | very weak |
| 3.04 | strong |
| 3.01 | weak |

**3.** The crystalline form according to claim 1 in substantially pure form.

**4.** The crystalline form according to claim 2 in substantially pure form.

**5.** A pharmaceutical preparation comprising 4-(5,6,7,8-tetrahydroimidazo[1,5-a]pyridin-5-yl)-benzonitrile hydrochloride in a crystalline form according to claim 1 or 2 and at least one pharmaceutically acceptable carrier.

**6.** The crystalline form according to any one of claims 1 to 4 for use in a method of therapeutically treating the animal or human body.

**7.** The crystalline form according to any one of claims 1 to 4 for use in the treatment of diseases that respond to inhibition of the enzyme aromatase.

**8.** The use of a crystalline form according to any one of claims 1 to 4 for the manufacture of a pharmaceutical preparation.

**9.** The use of a crystalline form according to any one of claims 1 to 4 for the manufacture of a pharmaceutical preparation for the treatment of diseases that respond to inhibition of the enzyme

aromatase.

**10.** A process for the preparation of a crystalline form according to any one of claims 1 to 4, which comprises

(a) reacting 4-(5,6,7,8-tetrahydroimidazo[1,5-a]pyridin-5-yl)-benzonitrile with hydrogen chloride in the presence of water, or

(b) treating with water a form of 4-(5,6,7,8-tetrahydroimidazo[1,5-a]pyridin-5-yl)-benzonitrile hydrochloride that contains less water than does the hemihydrate of formula I to be prepared, or

(c) removing water from a form of 4-(5,6,7,8-tetrahydroimidazo[1,5-a]pyridin-5-yl)-benzonitrile hydrochloride that contains more water than does the hemihydrate of formula I to be prepared, or

(d) converting a salt of 4-(5,6,7,8-tetrahydroimidazo[1,5-a]pyridin-5-yl)-benzonitrile other than the hydrochloride into the hydrochloride hemihydrate of formula I in the presence of water.

**Claims for the following Contracting States : ES, GR**

**1.** A process for the preparation of 4-(5,6,7,8-tetrahydroimidazo[1,5-a]pyridin-5-yl)-benzonitrile hydrochloride in crystalline hemihydrate form, which process comprises

(a) reacting 4-(5,6,7,8-tetrahydroimidazo[1,5-a]pyridin-5-yl)-benzonitrile with hydrogen chloride in the presence of water, or

(b) treating with water a form of 4-(5,6,7,8-tetrahydroimidazo[1,5-a]pyridin-5-yl)-benzonitrile hydrochloride that contains less water than does the hemihydrate of formula I to be prepared, or

(c) removing water from a form of 4-(5,6,7,8-tetrahydroimidazo[1,5-a]pyridin-5-yl)-benzonitrile hydrochloride that contains more water than does the hemihydrate of formula I to be prepared, or

(d) converting a salt of 4-(5,6,7,8-tetrahydroimidazo[1,5-a]pyridin-5-yl)-benzonitrile other than the hydrochloride into the hydrochloride hemihydrate of formula I in the presence of water.

**2.** A process according to claim 1 for the preparation of 4-(5,6,7,8-tetrahydroimidazo[1,5-a]pyridin-5-yl)-benzonitrile hydrochloride in crystalline hemihydrate form, the X-ray powder pattern of which (Guinier camera, copper-$K_{\alpha 1}$ radiation) exhibits the following lattice spacings (d values) and relative line intensities (intensity):

| d values [in Angstrom (≙ $10^{-10}$ m)] | Intensity |
|---|---|
| 13.4 | very strong |
| 9.5 | medium |
| 8.2 | weak |
| 7.5 | strong |
| 6.7 | very weak |
| 6.5 | very strong |
| 5.65 | very strong |
| 5.42 | strong |
| 5.34 | very strong |
| 5.15 | medium |
| 4.99 | medium |
| 4.84 | strong |
| 4.60 | strong |
| 4.51 | strong |
| 4.20 | strong |
| 4.09 | medium |
| 4.00 | strong |
| 3.83 | strong |
| 3.81 | medium |
| 3.76 | weak |
| 3.74 | medium |
| 3.66 | medium |
| 3.62 | strong |
| 3.56 | medium |
| 3.51 | medium |
| 3.40 | very weak |
| 3.35 | medium |
| 3.29 | very weak |
| 3.25 | strong |
| 3.21 | weak |
| 3.19 | weak |
| 3.15 | medium |
| 3.13 | very weak |
| 3.04 | strong |
| 3.01 | weak |

**3.** A process according to claim 1 for the preparation of the crystalline form according to claim 1 in substantially pure form.

**4.** A process according to claim 2 for the preparation of the crystalline form according to claim 2 in substantially pure form.

**Revendications**
**Revendications pour les Etats contractants suivants : AT, BE, CH, DE, DK, FR, GB, IT, LI, LU, NL, SE**

**1.** 4-(5,6,7,8-tétrahydroimidazo[1,5-a]pyridin-5-yl)-benzonitril-chlorhydrate sous forme cristalline d'hémihydrate.

**2.** 4-(5,6,7,8-tétrahydroimidazo[1,5-a]pyridin-5-yl)-benzonitril-chlorhydrate sous forme cristalline d'hémihydrate selon la revendication 1, caractérisé par les paramètres de réseau suivants (valeurs d) et les intensités relatives de raies (intensités) au diagramme de poudre de rayons X (caméra de Guinier, rayonnement $K_{\alpha 1}$ du cuivre) :

| Valeurs d [en Angstroem ($\cong 10^{-10}$ m)] | Intensité |
|---|---|
| 13,4 | très forte |
| 9,5 | moyenne |
| 8,2 | faible |
| 7,5 | forte |
| 6,7 | très faible |
| 6,5 | très forte |
| 5,65 | très forte |
| 5,42 | forte |
| 5,34 | très forte |
| 5,15 | moyenne |
| 4,99 | moyenne |
| 4,84 | forte |
| 4,60 | forte |
| 4,51 | forte |
| 4,20 | forte |
| 4,09 | moyenne |
| 4,00 | forte |
| 3,83 | forte |
| 3,81 | moyenne |
| 3,76 | faible |
| 3,74 | moyenne |
| 3,66 | moyenne |
| 3,62 | forte |
| 3,56 | moyenne |
| 3,51 | moyenne |
| 3,40 | très faible |
| 3,35 | moyenne |
| 3,29 | très faible |
| 3,25 | forte |
| 3,21 | faible |
| 3,19 | faible |
| 3,15 | moyenne |
| 3,13 | très faible |
| 3,04 | forte |
| 3,01 | faible |

**3.** Forme cristalline selon la revendication 1, sous une forme essentiellement pure.

**4.** Forme cristalline selon la revendication 2, sous une forme essentiellement pure.

**5.** Préparation pharmaceutique contenant du 4-(5,6,7,8-tétrahydroimidazo[1,5-a]pyridin-5-yl)benzonitril-chlorhydrate sous une forme cristalline selon la revendication 1 ou 2 et au moins un matériau véhicule pharmaceutiquement utilisable.

**6.** Forme cristalline selon l'une des revendications 1 à 4 pour utilisation dans un procédé de traitement thérapeutique du corps humain ou animal.

**7.** Forme cristalline selon l'une des revendications 1 à 4, pour utilisation dans le traitement des maladies qui répondent à une inhibition de l'enzyme aromatase.

**8.** Utilisation d'une forme cristalline selon l'une des revendications 1 à 4 pour produire des préparations pharmaceutiques.

**9.** Utilisation d'une forme cristalline selon l'une des revendications 1 à 4 pour produire des préparations pharmaceutiques pour le traitement de maladies qui répondent à une inhibition de l'enzyme aromatase.

10. Procédé de préparation d'une forme cristalline selon l'une des revendications 1 à 4, caractérisé en ce que
    (a) on fait réagir le 4-(5,6,7,8-tétrahydroimidazo[1,5-a]pyridin-5-yl)-benzonitrile avec de l'acide chlorhydrique en présence d'eau, ou
    (b) on traite une forme cristalline du 4-(5,6,7,8-tétrahydroimidazo[1,5-a]pyridin-5-yl)benzonitril-chlorhydrate, qui contient moins d'eau que l'hémihydrate de formule I à préparer, avec de l'eau, ou
    (c) on élimine de l'eau d'une forme de cristallisation du 4-(5,6,7,8-tétrahydroimidazo[1,5-a]pyridin-5-yl)-benzonitril-chlorhydrate, qui contient plus d'eau que l'hémihydrate de formule I à préparer, ou
    (d) on transforme un sel différent du chlorhydrate du 4-(5,6,7,8-tétrahydroimidazo[1,5-a]pyridin-5-yl)-benzonitrile en présence d'eau en le chlorhydrate-hémihydrate de formule I.

**Revendications pour les Etats contractants suivants : ES, GR**

1. Procédé de préparation du 4-(5,6,7,8-tétrahydroimidazo[1,5-a]pyridin-5-yl)-benzonitrilchlorhydrate sous forme cristalline hémihydrate, caractérisé en ce que
    (a) on fait réagir le 4-(5,6,7,8-tétrahydromidazo[1,5-a]pyridin-5-yl)-benzonitrile avec de l'acide chlorhydrique en présence d'eau, ou
    (b) on traite une forme cristalline du 4-(5,6,7,8-tétrahydroimidazo[1,5-a]pyridin-5-yl)-benzonitril-chlorhydrate, qui contient moins d'eau que l'hémihydrate de formule I à préparer, avec de l'eau, ou
    (c) on élimine de l'eau d'une forme de cristallisation du 4-(5,6,7,8-tétrahydroimidazo[1,5-a]pyridin-5-yl)-benzonitril-chlorhydrate, qui contient plus d'eau que l'hémihydrate de formule I à préparer, ou
    (d) on transforme un sel différent du chlorhydrate du 4-(5,6,7,8-tétrahydroimidazo[1,5-a]pyridin-5-yl)-benzonitrile en présence d'eau en le chlorhydrate-hémihydrate de formule I.

2. Procédé selon la revendication 1 de préparation du 4-(5,6,7,8-tétrahydroimidazo[1,5-a]pyridin-5-yl)-benzonitril-chlorhydrate sous forme cristalline hémihydrate qui a les paramètres de réseau suivants (valeurs d) et les intensités relatives de raies (intensités) du diagramme de poudre de rayons X (caméra de Guinier, rayonnement $K_{\alpha 1}$ du cuivre) :

| Valeurs d [en Angstroem ($\cong 10^{-10}$ m)] | Intensité |
|---|---|
| 13,4 | très forte |
| 9,5 | moyenne |
| 8,2 | faible |
| 7,5 | forte |
| 6,7 | très faible |
| 6,5 | très forte |
| 5,65 | très forte |
| 5,42 | forte |
| 5,34 | très forte |
| 5,15 | moyenne |
| 4,99 | moyenne |
| 4,84 | forte |
| 4,60 | forte |
| 4,51 | forte |
| 4,20 | forte |
| 4,09 | moyenne |
| 4,00 | forte |
| 3,83 | forte |
| 3,81 | moyenne |
| 3,76 | faible |
| 3,74 | moyenne |
| 3,66 | moyenne |
| 3,62 | forte |
| 3,56 | moyenne |
| 3,51 | moyenne |
| 3,40 | très faible |
| 3,35 | moyenne |
| 3,29 | très faible |
| 3,25 | forte |
| 3,21 | faible |
| 3,19 | faible |
| 3,15 | moyenne |
| 3,13 | très faible |
| 3,04 | forte |
| 3,01 | faible |

**3.** Procédé selon la revendication 1 de préparation de la forme cristalline selon la revendication 1, sous forme essentiellement pure.

**4.** Procédé selon la revendication 2 de préparation de la forme cristalline selon la revendication 2, sous forme essentiellement pure.